Europäisches Patentamt

European Patent Office

Office européen des brevets

(19)

(11) **EP 0 900 591 B1**

(12) **EUROPÄISCHE PATENTSCHRIFT**

(45) Veröffentlichungstag und Bekanntmachung des Hinweises auf die Patenterteilung:
**23.10.2002 Patentblatt 2002/43**

(51) Int Cl.[7]: **B01J 19/08**, B01J 35/04

(21) Anmeldenummer: **98810849.4**

(22) Anmeldetag: **27.08.1998**

(54) **Entladungsreaktor**

Electric discharge reactor

Réacteur à décharge électrique

(84) Benannte Vertragsstaaten:
**CH DE FR GB IT LI NL SE**

(30) Priorität: **08.09.1997 DE 19739181**

(43) Veröffentlichungstag der Anmeldung:
**10.03.1999 Patentblatt 1999/10**

(73) Patentinhaber: **ABB RESEARCH LTD.**
**8050 Zürich (CH)**

(72) Erfinder:
• **Eliasson, Baldur, Dr.**
**5413 Birmenstorf (CH)**

• **Kogelschatz, Ulrich, Dr.**
**5212 Hausen (CH)**

(74) Vertreter: **ABB Patent Attorneys**
**c/o ABB Schweiz AG**
**Brown Boveri Strasse 6**
**5400 Baden (CH)**

(56) Entgegenhaltungen:
WO-A-91/16528        DE-A- 4 220 865
DE-A- 4 416 676        US-A- 3 674 666
US-A- 4 737 885        US-A- 4 954 320
US-A- 5 609 736

## Beschreibung

## Technisches Gebiet

Die Erfindung betrifft einen Entladungsreaktor sowie Verwendungen desselben. Bekannte gattungsgemässe Entladungsreaktoren für stille Entladungen und ähnliche Vorgänge weisen zwischen zwei Elektroden mindestens ein Dielektrikum und einen Gas enthaltenden Entladungsspalt auf und sind an eine Wechselspannungsquelle angeschlossen, meist zu mehreren parallel. Bei jeder Halbwelle werden im Spalt Mikroentladungen gezündet, durch das Dielektrikum begrenzte Durchschläge, welche freie Radikale erzeugen und dadurch bestimmte chemische Umsetzungen im Gas auslösen.

## Stand der Technik

Allgemeine Informationen zu stillen Entladungen und ihren Anwendungen sind z. B. den folgenden Veröffentlichungen zu entnehmen: U. Kogelschatz: 'Silent Discharges and their Applications' in: 'Proceedings of the Tenth International Conference on Gas Discharges and their Applications', Vol. II, Swansea 1992 und B. Eliasson, U. Kogelschatz: 'Nonequilibrium Volume Plasma Chemical Processing', IEEE Transactions on Plasma Science 19/6, S. 1063-1077 (1991).

Seit langem werden Entladungsreaktoren zur Herstellung von Ozon aus $O_2$ oder Luft für die Trinkwasseraufbereitung und andere Zwecke verwendet. Ausser auf die obengenannten Veröffentlichungen wird in diesem Zusammenhang hingewiesen auf U. Kogelschatz, B. Eliasson: 'Ozone Generation and Applications' in J. -S. Chang, A. J. Kelly, J. M. Crowley: 'Handbook of Electrostatic Processes', Marcel Dekker, Inc. (1995) und auf DE-C-32 20 018.

Eine weitere Anwendung, die im Hinblick auf die immer dringlicher werdende Verringerung des Ausstosses von Treibhausgasen von grossem Interesse ist, ist die Umsetzung von $CO_2$ und $H_2$ in Methanol und Wasser, s. A. Bill, A. Wokaun, B. Eliasson, E. Killer, U. Kogelschatz: 'Greenhouse Gas Chemistry', Energy Convers. Mgmt. 38, Suppl., S. 415-422 (1997) und J. U. Höltje: 'Untersuchung der Makrokinetik der heterogen katalysierten Synthese aus Kohlendioxid und Wasserstoff zu Methanol', Dissertation, Rheinisch-Westfälische Technische Hochschule, Aachen 1991. Auch die gemeinsame Umsetzung der Treibhausgase $CO_2$ und $CH_4$ zu Synthesegas oder Syngas, einer Mischung aus CO und $H_2$, ist in diesem Zusammenhang von Bedeutung.

Weitere Anwendungen sind die Zersetzung von Schadgasen z. B. in Rauchgasen aus Müllverbrennungsanlagen, aber auch in den Abgasen von Kraftfahrzeugen (s. DE-C-195 18 970) sowie Excimerlampen, die in einem engen Frequenzband liegende UV-Strahlung liefern, welche beim Zerfall angeregter Zustände von Edelgasatomen entsteht (s. z. B. EP-B-0 547 366).

Entladungsreaktoren sind in verschiedenen Ausführungen bekannt. So können die Elektroden z. B. als parallele Platten oder als konzentrische Rohre ausgebildet sein. Durchwegs wird ein entsprechend geformtes, die Elektroden durchgehend trennendes Dielektrikum eingesetzt, das aus mindestens einer Schicht von massivem nichtleitendem Material, z. B. Glas besteht. Sie kann unmittelbar an einer Elektrode angeordnet oder auch von beiden Elektroden beabstandet sein. Es ist auch möglich, zwei derartige Schichten, vorzugsweise jeweils anschliessend an die Elektroden, anzuordnen. Im Raum zwischen den Elektroden liegt jeweils mindestens ein Entladungsspalt, in welchen die gasförmigen Edukte der gewünschten chemischen Umsetzung eingeleitet werden und in welchem sich unter dem Einfluss des zwischen den Elektroden aufgebauten elektrischen Feldes Mikroentladungen bilden, bei denen hochreaktive Zwischenprodukte entstehen, freie Elektronen und Radikale, aus deren Reaktionen untereinander und vor allem mit Gasmolekülen oder -atomen mit einer von verschiedenen Randbedingungen abhängigen Ausbeute die gewünschten Produkte hervorgehen.

Die zwischen die Elektroden gelegte Speisespannung kann wie bei den frühen Ozonerzeugungsanlagen der Netzfrequenz entsprechen, bei den heutigen Anlagen liegt jedoch die Frequenz im Interesse einer möglichst hohen Ausbeute gewöhnlich wesentlich höher und kann bis in den GHz-Bereich gehen.

Die Leistungsaufnahme P des Gases bei der stillen Entladung folgt dem Gesetz

$$(1) \qquad P = 4fC_D \ U_B \ (\hat{U} - (1-\beta)U_B),$$

wobei f die Frequenz der Speisespannung, $\hat{U}$ ihre Amplitude, $C_D$ die Kapazität des Dielektrikums, $U_B$ die mittlere Brennspannung der Mikroentladungen und

$$(2) \qquad \beta = C_S/C_D$$

den Quotienten aus der Kapazität $C_S$ des Entladungsspalts und der Kapazität $C_D$ des Dielektrikums bedeutet.

Bei festen Werten für die Frequenz f, die Amplitude $\hat{U}$ und die Kapazität des Dielektrikums $C_D$ hängt die Leistungsaufnahme somit von $U_B$ und $\beta$ ab, welche ihrerseits von der Spaltweite des Entladungsspalts d abhängen. Bei sonst gegebenen Randbedingungen (Gaszusammensetzung, Druck und Temperatur im Entladungsspalt) lässt sich die Leistungsaufnahme P und mit ihr die Ausbeute des Entladungsreaktors somit durch Einstellung dieser Grösse optimieren. Tatsächlich ist jedoch die optimale Weite des Entladungsspaltes meist so klein, dass ihre Einstellung bei für eine wirtschaftliche Produktion ausreichend leistungsfähigen und entsprechend grossen Entladungsreaktoren an durch Herstellungstoleranzen gegebene Grenzen stösst und die tatsächliche Spaltweite mehr oder weniger weit über dem Optimum liegt.

Es ist zwar bekannt, den Entladungsspalt mit einem Material auszufüllen, welches einen Teil des Volumens einnimmt und ein zusammenhängendes verästeltes Gasvolumen freilässt. So sind in der JP-A-103 903/89, der JP-A-038 881/96, der JP-A-261 034/89 und der US-A-5 254 231 bei gattungsgemässen oder ähnlichen Reaktoren den Entladungsspalt ausfüllende Schüttungen von Partikeln aus z. B. Keramik beschrieben. Bei derartigen Schüttungen entstehen jedoch Hohlräume sehr unterschiedlicher und kaum kontrollierbarer Grösse und die Porosität ist im Ganzen gering, so dass das Volumen schlecht ausgenutzt wird und der Strömungswiderstand für das durchströmende Gas hoch ist. Die Granulate sind auch umständlich in der Handhabung und ihre Eigenschaften können leicht durch mechanische Einwirkungen beeinträchtigt werden.

Aus der DE-A-42 20 865 ist ein gattungsgemässer Entladungsreaktor bekannt, bei welchem der Entladungsspalt mit Glas-, Quarz- oder Mineralwolle ausgefüllt ist. Diese Materialien lassen jedoch ebenfalls Hohlräume unterschiedlicher und schwer kontrollierbarer Ausdehnung frei. Eine lückenlose Ausfüllung des Entladungsspaltes ist wegen ihrer mechanischen Eigenschaften nur schwer zu erreichen. In der gleichen Schrift ist auch die Möglichkeit erwähnt, auf einer der Elektroden eine poröse Schicht anzulegen. Es handelt sich dabei jedoch lediglich um eine verhältnismässig dünne Schicht aus einem Katalysatormaterial oder aus einem Trägermaterial für ein solches, welches nur einen kleinen Teil des Entladungsspalts einnimmt.

In der DE 44 16 676 A1 ist eine Vorrichtung zur Entgiftung von Abgasen aus mobilen Anlagen mit einem nach dem Prinzip der stillen Entladung arbeitenden Plasmareaktor offenbart. Letzterer umfasst einen Isolierstoffkörper, welcher den Raum zwischen zwei benachbarten Elektroden vollständig ausfüllt und aus einem offenporigen Material oder einem Bündel von keramischen Isolierstofffasern besteht.

## Darstellung der Erfindug

Der Erfindung liegt die Aufgabe zugrunde, einen gattungsgemässen Entladungsreaktor mit verbessertem Wirkungsgrad zu schaffen, welcher einfach herzustellen und mechanisch stabil ist Diese Aufgabe wird durch die Merkmale des Anspruchs 1 gelöst.

Die Erfindung schafft einen Entladungsreaktor, bei welchem auch bei grossen Abmessungen ohne weiteres kleine effektive Spaltweiten erzielbar sind, wobei durch das verwendete poröse Material der Entladungsspalt stabil in eine Vielzahl von verhältnismässig gut definierten Volumina, nämlich die einzelnen Poren aufgeteilt wird, in welchen sich jeweils voneinander weitgehend unabhängige Mikroentladungen bilden, so dass die für den Ablauf der stillen Entladung massgebende effektive Spaltweite d der lichten Weite der Poren entspricht. Auf diese Weise kann eine wesentlich höhere Leistungsaufnahme erzielt und die Ausbeute deutlich verbessert werden. Die Porosität kann sehr hoch gewählt werden, was den Wirkungsgrad verbessert und den Strömungswiderstand vermindert.

Die erfindungsgemässe Ausbildung gattungsgemässer Ent.ladungsreaktoren hat jedoch noch weitere Vorteile. So ist die Ableitung der von den Mikroentladungen erzeugten Wärme aus dem Entladungsspalt durch Wärmeleitung über das Gerüst des offenporigen Materials wesentlich wirksamer als diejenige über das Gas oder über bekannte zur Auffüllung des Entladungsspalts verwendete Materialien. Es ist daher wesentlich leichter, das Gas auf einer annähernd optimalen Temperatur zu halten.

Zudem steht eine grosse Fläche zur Verfügung, welche für die Auslösung der gewünschten chemischen Umsetzung oder die Verbesserung ihrer Ausbeute nutzbar gemacht werden kann, indem das Gerüst mit einem geeigneten Katalysator beschichtet oder versetzt wird.

## Kurze Beschreibung der Zeichnungen

Im folgenden wird die Erfindung anhand von Figuren, welche lediglich Ausführungsbeispiele zeigen, näher erläutert. Es zeigen

Fig. 1     eine schematische Darstellung eines erfindungsgemässen Entladungsreaktors gemäss einer ersten Ausführungsform,

Fig. 2     eine schematische Darstellung eines erfindungsgemässen Entladungsreaktors gemäss einer zweiten Ausführungsform und

Fig.3     die Oberfläche eines Blocks von für die Verwendung in erfindungsgemässen Entladungsreaktoren geeignetem offenporigem Material.

## Wege zur Ausführung der Erfindung

Der Entladungsreaktor weist jeweils eine erste Elektrode 1 und eine zweite Elektrode 2 auf, an die eine Speisespannungsquelle 3 gelegt ist. Die Elektroden 1, 2 können verschiedene geometrische Formen aufweisen. Insbesondere können sie als parallele beabstandete Platten oder als vorzugsweise konzentrisch angeordnete Rohre ausgebildet sein. Vor allem die letztere Konfiguration wird gern gewählt, wobei dann eine Vielzahl von Entladungsreaktoren parallel zu einer wabenartig aufgebauten, zwischen den Reaktoren von Kühlwasser durchströmten Batterie zusammengefasst und auch elektrisch parallelgeschaltet ist. Mit einer derartigen Anordnung kann auf kleinem Raum eine hohe Produktionsleistung erzielt werden.

Die Speisespannungsquelle 3 liefert eine Wechselspannung, deren Frequenz zwischen einigen Hz und einigen GHz liegen kann. Da nach (1) die Leistungsaufnahme des Entladungsreaktors innerhalb gewisser Grenzen proportional zur Frequenz ist, werden heute im Interesse einer hohen Ausbeute im allgemeinen hohe, im MHz- oder sogar GHz-Bereich liegende Frequenzen bevorzugt. Die Amplitude muss so gross sein, dass im Entladungsspalt die Zündspannung erreicht und bei jeder Halbwelle Mikroentladungen ausgelöst werden.

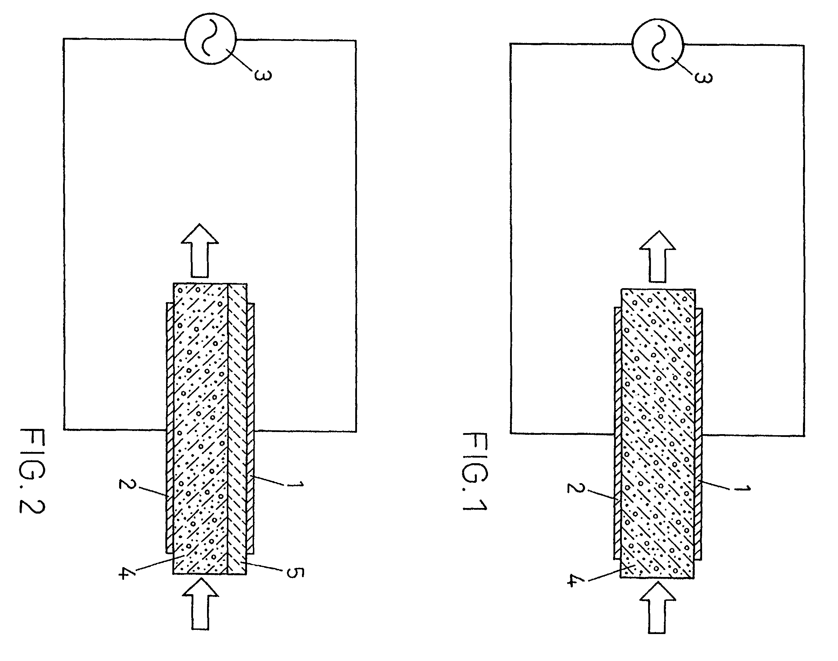

Der zwischen der ersten Elektrode 1 und der zweiten Elektrode 2 liegende Spalt ist beim Entladungsreaktor gemäss der ersten Ausführungsform (Fig. 1) ganz von einer Schicht aus einem Füllstoff ausgefüllt, einem starren offenporigen Material, welches auch als die Mikroentladungen begrenzendes Dielektrikum wirkt. Das starre offenporige Material ist in Form eines festen Blocks 4 zwischen den Elektroden angeordnet.

Zwischen den Elektroden 1, 2 wird ein Gasstrom (Pfeile) durchgeleitet, in welchem durch die in den Poren des Blocks 4 ablaufende stille Entladung die gewünschten chemischen Umsetzungen ausgelöst werden. Das aus dem Entladungsspalt abgezogene Gas kann dann mit physikalischen oder chemischen Methoden weiterverarbeitet und z. B. die gewünschten Produkte isoliert werden. Die chemischen Umsetzungen können u. U. durch Katalysatoren im Block 4 unterstützt oder überhaupt erst ermöglicht werden. Statt parallel zu den Elektroden kann der Gasstrom auch durch die Elektroden geführt werden, die dann natürlich gasdurchlässig ausgebildet sein müssen, z. B. als poröse Sinterplatten oder perforierte Metallmembranen oder -platten.

Obwohl der Block 4 als Schutz gegen Durchschläge in der Regel ausreicht, kann, wie bei der zweiten Ausführungsform gemäss Fig. 2 dargestellt, zur diesbezüglichen Sicherung das Dielektrikum durch eine zwischen den Elektroden 1, 2 durchgehend angeordnete Sperrschicht 5 aus massivem Material verstärkt sein. Es kann sich dabei z. B. um Glas, Quarz, Keramik oder ein anderes geeignetes nichtleitendes Material handeln. Die Schicht kann direkt an einer der Elektroden, z. B. wie dargestellt an der ersten Elektrode 1 oder auch von beiden Elektroden beabstandet angeordnet sein.

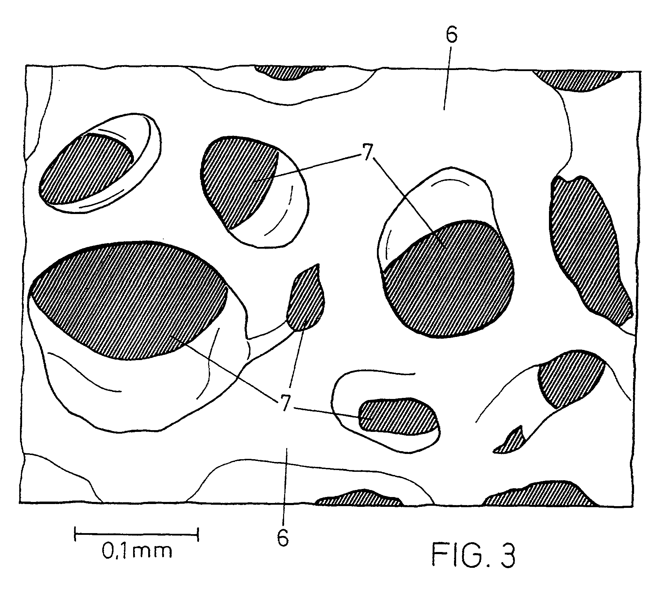

Das starre offenporige Material des Blocks 4 weist ein festes Gerüst 6 (Fig. 3) auf, das das Volumen des Entladungsspalts in eine Vielzahl offener, miteinander verbundener Poren 7 abteilt. Das Gerüst muss aus nichtleitendem Material bestehen, das gegenüber Mikroentladungen resistent und chemisch inert ist. In letzterer Hinsicht hängen die spezifischen Anforderungen vom Anwendungsgebiet ab. Die optimale Porengrösse ist, wie sich aus dem weiter oben im Zusammenhang mit der Leistungsaufnahme im Entladungsspalt Auseinandergesetzten ergibt, von den geometrischen und elektrischen Randbedingungen abhängig. In der Regel dürfte sie in der Grössenordnung von 0,1 mm, etwa zwischen 0,05 mm und 0,2 mm liegen. Günstig im Sinne einer hohen Ausbeute sind auch ein grosses Reaktionsvolumen, d. h. eine hohe Porosität von mindestens 50%, vorzugsweise 80-90% oder mehr und eine hohe Dielektrizitätskonstante, die etwa zwischen 3 und 20 liegen kann. Die erwünschte Stabilisierung der Gastemperatur wird durch hohe Wärmeleitfähigkeit und Wärmekapazität begünstigt.

Diese Anforderungen werden von verschiedenen bekannten Stoffen erfüllt, insbesondere von porösem Glas und porösem Quarz, der sich wegen seiner UV-Durchlässigkeit auch für die Anwendung in Excimer-Lampen eignet. Sehr günstig ist auch poröse Keramik, wie sie von der Firma Bridgestone unter der Bezeichnung Ceramic Foam angeboten wird (Ceramic Foam, Technical Report No. 1). Dieses Material zeichnet sich durch hohe Porosität (80-90%), niedriges spezifisches Gewicht, hohe Hitzebeständigkeit (bis 1'150°C) und chemische Resistenz aus. Ausserdem ist der Druckabfall des Gasstroms verhältnismässig gering. Auch die Verwendung von porösem keramischem Sintermaterial ist möglich.

Alle erwähnten Materialien sind mechanisch sehr stabil, isbesondere sind ihre funktionswichtigen Eigenschaften mechanischen Belastungen gegenüber robust. Die Porengrösse ist in der Regel gut definiert und schwankt verhältnismässig wenig.

Erfindungsgemässe Entladungsreaktoren eignen sich praktisch für alle bekannten Anwendungen bekannter gattungsgemässer Reaktoren. Insbesondere ist dabei an die Ozonerzeugung zu denken sowie an die Erzeugung von Methanol aus $CO_2$ und $H_2$ und von Synthesegas (CO und $H_2$) aus $CO_2$ und $CH_4$. Zur Erzeugung von Methanol werden $CO_2$ und $H_2$ durch den Entladungsreaktor geleitet und unter der Einwirkung der stillen Entladung z. T. zu Methanol und Wasser umgesetzt. Bei dieser Reaktion werden $Cu/ZrO_2$- oder $Cu/ZnO$-Katalysatoren eingesetzt. Bei der im übrigen analogen Erzeugung von Synthesegas können z. B. Nickelverbindungen als Katalysatoren dienen.

Beim erfindungsgemässen Entladungsreaktor kann nun das Gerüst 6 des Blocks 4 mit Katalysatormaterial beschichtet oder versetzt sein. Zur Beschichtung können z. B. feste Kupfer- und Zinkverbindungen usw. in einem Lösungsmittel gelöst, dann zusammen mit demselben verdampft und aus der Gasphase an den Wänden der Poren 7 abgeschieden werden. Auch Abscheidung unmittelbar aus gasförmigen metallorganischen Verbindungen ist möglich. Die Abscheidung kann durch Anheben der Temperatur oder eventuell zusätzlich durch Zünden einer stillen Entladung ausgelöst werden. Es kann dann ein Oxidationsschritt folgen, bei dem das Material des Blocks 4 Luft oder Sauerstoff ausgesetzt wird und Konditionierung in einer wasserstoffhaltigen Atmosphäre. Auch bei diesen Schritten können Temperaturbehandlung und stille Entladung eingesetzt werden.

**Bezugszeichenliste**

1    erste Elektrode
2    zweite Elektrode
3    Speisespannungsquelle
4    Block aus starrem offenporigem Material
5    Sperrschicht aus massivem Material
6    Gerüst
7    Poren

**Patentansprüche**

1. Entladungreaktor mit mindestens einer ersten Elektrode (1) und einer von derselben beabstandeten zweiten Elektrode (2) und einem zwischen denselben liegenden Entladungsspalt, welcher von einem Dielektrikum ausgefüllt ist, das eine Schicht eines Füllstoffs umfasst, welcher ein eine Vielzahl miteinander verbundener Poren (7) bildendes Gerüst (6) aufweist, **dadurch gekennzeichnet, dass** der Füllstoff als mindestens ein Block (4) eines starren offenporigen Materials mit einer Porosität von mindestens 50% ausgebildet ist.

2. Entladungsreaktor nach Anspruch 1, **dadurch gekennzeichnet, dass** die lichte Weite der Poren (7) des Blocks (4) zwischen 0,05 mm und 0,2 mm liegt.

3. Entladungsreaktor nach Anspruch 1 oder 2, **dadurch gekennzeichnet, dass** die Porosität des Blockes (4) zwischen 80% und 95% liegt.

4. Entladungsreaktor nach einem der Ansprüche 1 bis 3, **dadurch gekennzeichnet, dass** das Material des Gerüstes (6) des Blockes (4) eine Dielektrizitätskonstante zwischen 3 und 20 aufweist.

5. Entladungsreaktor nach einem der Ansprüche 1 bis 4, **dadurch gekennzeichnet, dass** das Gerüst (6) des Blockes (4) aus Keramik, Glas oder Quarz besteht.

6. Entladungsreaktor nach einem der Ansprüche 1 bis 5, **dadurch gekennzeichnet, dass** das Dielektrikum mindestens eine Sperrschicht (5) aus massivem Material enthält.

7. Entladungsreaktor nach einem der Ansprüche 1 bis 6, **dadurch gekennzeichnet, dass** das Gerüst (6) des Blocks (4) mit einem Katalysatormaterial beschichtet oder versetzt ist.

8. Entladungsreaktor nach Anspruch 7, **dadurch gekennzeichnet, dass** das Katalysatormaterial mindestens eine Metallverbindung enthält.

9. Entladungsreaktor nach Anspruch 8, **dadurch gekennzeichnet, dass** das Katalysatormaterial Cu und $ZrO_2$ oder Cu und ZnO enthält.

10. Entladungsreaktor nach Anspruch 8, **dadurch gekennzeichnet, dass** das Katalysatormaterial mindestens eine Nickelverbindung enthält.

**Claims**

1. Discharge reactor with at least one first electrode (1) and one second electrode (2) spaced from the latter and with a discharge gap which is located between them and which is filled up by a dielectric comprising a layer of a filler which has a skeleton (6) forming a multiplicity of interconnected pores (7), **characterized in that** the filler is designed as at least one block (4) of a rigid open-pored material having a porosity of at least 50%.

2. Discharge reactor according to Claim 1, **characterized in that** the clear width of the pores (7) of the block (4) is between 0.05 mm and 0.2 mm.

3. Discharge reactor according to claim 1 or 2, **characterized in that** the porosity of the block (4) is between 80% and 90%.

4. Discharge reactor according to one of Claims 1 to 3, **characterized in that** the material of the skeleton (6) of the block (4) has a dielectric constant of between 3 and 20.

5. Discharge reactor according to one of Claims 1 to 4, **characterized in that** the skeleton (6) of the block (4) consists of ceramic, glass or quartz.

6. Discharge reactor according to one of Claims 1 to 5, **characterized in that** the dielectric contains at least one barrier layer (5) consisting of solid material.

7. Discharge reactor according to one of Claims 1 to 6, **characterized in that** the skeleton (6) of the block (4) is coated or packed with a catalyst material.

8. Discharge reactor according to Claim 7, **characterized in that** the catalyst material contains at least one metal compound.

9. Discharge reactor according to Claim 8, **characterized in that** the catalyst material contains Cu and $ZrO_2$ or Cu and ZnO.

10. Discharge reactor according to Claim 8, **characterized in that** the catalyst material contains at least one nickel compound.

**Revendications**

1. Réacteur à décharge électrique avec au moins une première électrode (1) et une seconde électrode (2) distante de celle-ci et un intervalle de décharge situé entre celles-ci, qui est rempli d'un diélectrique, qui comprend une couche d'un matériau de remplissage qui présente une structure (6) formant une pluralité de pores (7) communiquant les uns avec les autres, **caractérisé en ce que** le matériau de remplissage est configuré en au moins un bloc (4) d'un matériau rigide à porosité ouverte, avec une porosité d'au moins 50 %.

2. Réacteur à décharge électrique suivant la revendication 1, **caractérisé en ce que** la largeur libre des pores (7) du bloc (4) est comprise entre 0,05 mm et 0,2 mm.

3. Réacteur à décharge électrique suivant la revendication 1 ou 2, **caractérisé en ce que** la porosité du bloc (4) est comprise entre 80 % et 90 %.

4. Réacteur à décharge électrique suivant l'une quelconque des revendications 1 à 3, **caractérisé en ce que** le matériau de la structure (6) du bloc (4) présente une constante diélectrique comprise entre 3 et 20.

5. Réacteur à décharge électrique suivant l'une quelconque des revendications 1 à 4, **caractérisé en ce que** la structure (6) du bloc (4) se compose de céramique, de verre ou de quartz.

6. Réacteur à décharge électrique suivant l'une quelconque des revendications 1 à 5, **caractérisé en ce que** le diélectrique comporte au moins une couche de barrage (5) en un matériau massif.

7. Réacteur à décharge électrique suivant l'une quelconque des revendications 1 à 6, **caractérisé en ce que** la structure (6) du bloc (4) est revêtue de ou remplacée par un matériau de catalyseur.

8. Réacteur à décharge électrique suivant la revendication 7, **caractérisé en ce que** le matériau de catalyseur comprend au moins un composé métallique.

9. Réacteur à décharge électrique suivant la revendication 8, **caractérisé en ce que** le matériau de catalyseur contient du Cu et du $ZrO_2$ ou du Cu et du ZnO.

10. Réacteur à décharge électrique suivant la revendication 8, **caractérisé en ce que** le matériau de catalyseur contient au moins un composé de nickel.

**EP 0 900 591 B1**

FIG.1

FIG.2

FIG. 3

0,1 mm